# EUROPEAN PATENT SPECIFICATION

(11) **EP 3 675 722 B1**
(45) Date of publication and mention of the grant of the patent: **17.07.2024**
(21) Application number: 18779761.8
(22) Date of filing: 27.08.2018
(51) Int. Cl.: A61B 5/024, A61B 5/05

(54) **HEART RATE DETECTION DEVICE AND RELATED METHOD**
HERZFREQUENZDETEKTIONSVORRICHTUNG UND ZUGEHÖRIGES VERFAHREN
DISPOSITIF DE DÉTECTION DE FRÉQUENCE CARDIAQUE ET PROCÉDÉ ASSOCIÉ

(30) Priority: 01.09.2017 US 201762553202 P
(43) Date of publication of application: 08.07.2020
(73) Proprietor: Société des Produits Nestlé S.A., 1800 Vevey (CH)
(72) Inventor: PEREZ-CAMARGO, Gerardo, House Springs, Missouri 63051 (US); NEUBARTH, Stuart Kyle, Sunnyvale, California 94089 (US); DONAVON, Mark Alan, Troy, Illinois 62294 (US)
(74) Representative: Elkington and Fife LLP
(86) International application number: PCT/IB2018/056515
(87) International publication number: WO 2019/043558

(56) References cited:
- EP-A1- 3 138 478
- WO-A2-2016/193972
- SU-A1- 625 130
- US-A1- 2017 027 344

## Description

### CROSS REFERENCE TO RELATED APPLICATIONS

This application claims priority to U.S. Provisional Application No. 62/553202 filed September 1, 2017.

### TECHNICAL FIELD

This disclosure relates generally to devices for detecting heart rate, heart rate variability, heart beat-to-beat intervals, respiration rate, and breath-to-breath intervals, and relates more particularly to heart rate devices with at least one capacitive displacement sensor.

### BACKGROUND

Similar to the trends seen in human health and fitness monitoring technologies, products are rapidly emerging within the companion animal category leveraging existing and emerging technologies to acquire an individual animal's biometric and behavioral data that can then be translated into custom insights in the animal's current and predictive health. Emotions like fear or happiness affect in a non-voluntary manner how fast the heart beats (Heart Rate or HR) and the rhythm of the heart (time between beats or Heart Rate Variability or HRV). Detecting changes in HR and HRV helps provide information about the changes in emotional status in pets. Involved pet owners want to know about the well-being of their pets when they cannot be together. Pet owners might want to have remote information about the emotional status of their pets while at work or during travel. Pet owner can also have access to the pet's wellbeing while the pet is at the groomers, in a boarding kennel, or with a dog walker. Additionally, changes in HR and respiration can help detect changes in normal cardiac function and respiration, these changes can alert the pet owner of the need to take the pet to the veterinarian. The ability to acquire heart rate and heart rate variability data on a person or animal can offer a rich source of data to identify both current and predictive mood and health insights custom to the individual. While current technologies have been seen that enable heart rate and heart rate variability signal acquisition with accuracy on companion animals when at rest, motion or movement by the animal introduces noise artifacts into these signals which significantly reduce the accuracy and confidence available in the data. Therefore, there is a need for an approach to heart rate and heart rate signal acquisition that can significantly increase the confidence and accuracy in the data beyond the capability of other existing technologies.

US 2017/0027344 discloses a system for alleviating airway blockage in a sleeping individual, the system comprising a capacitive proximity detector for detecting the user's heartrate.

WO 2016/193972 discloses a system for monitoring an object, including the heartrate of a human or animal, using an electric field sensor to measure the electrical capacitance between the sensor and a predetermined area of the object.

EP 3138478 discloses a wearable heartrate sensing module comprising a capacitive sensor for measuring the distance between the sensor and the user's skin.

### SUMMARY OF THE INVENTION

This disclosure relates generally to devices for detecting heart rate, heart rate variability, heart beat-to-beat intervals, respiration rate, and breath-to-breath intervals. The disclosure relates more particularly to heart rate devices with at least one capacitive displacement sensor.

A device in accordance with the invention for detecting a heart rate is provided according to claim 1

A system for determining a heart rate is provided according to claim 3.

A method in accordance with the invention for determining a heart rate is provided according to claim 7.

### BRIEF DESCRIPTION OF THE DRAWINGS

FIG. 1 illustrates a front elevation view of a computer system that is suitable for implementing at least part of a central computer system.
FIG. 2 illustrates a representative block diagram of exemplary elements included on the circuit boards inside a chassis of the computer system of FIG. 1.
FIG. 3 illustrates a representative block diagram of a system, according to an embodiment.
FIG. 4 illustrates a representative block diagram of a portion of the system of FIG. 3, according to an embodiment.
FIG. 5 illustrates a representative block diagram of another portion of the system of FIG. 3, according to an embodiment.
FIG. 6 illustrates is a flowchart for a method, according to an embodiment.
FIG. 7 illustrates is a flowchart for a method, according to an embodiment.
FIG. 8 illustrates a heart rate detecting device, according to an embodiment.
FIG. 9 illustrates graphs of a data associated with an embodiment.
FIG. 10 illustrates a heart rate detecting device, according to another embodiment.

### DETAILED DESCRIPTION OF THE INVENTION

For simplicity and clarity of illustration, the drawing figures illustrate the general manner of construction, and descriptions and details of well-known features and techniques may be omitted to avoid unnecessarily obscuring the present disclosure. Additionally, elements in the drawing figures are not necessarily drawn to scale. For example, the dimensions of some of the elements in the figures may be exaggerated relative to other elements to help improve understanding of embodiments of the present disclosure. The same reference numerals in different figures denote the same elements.

The terms "first," "second," "third," "fourth," and the like in the description and in the claims, if any, are used for distinguishing between similar elements and not necessarily for describing a particular sequential or chronological order. It is to be understood that the terms so used are interchangeable under appropriate circumstances such that the embodiments described herein are, for example, capable of operation in sequences other than those illustrated or otherwise described herein. Furthermore, the terms "include," and "have," and any variations thereof, are intended to cover a non-exclusive inclusion, such that a process, method, system, article, device, or apparatus that comprises a list of elements is not necessarily limited to those elements, but may include other elements not expressly listed or inherent to such process, method, system, article, device, or apparatus.

The terms "left," "right," "front," "back," "top," "bottom," "over," "under," and the like in the description and in the claims, if any, are used for descriptive purposes and not necessarily for describing permanent relative positions. It is to be understood that the terms so used are interchangeable under appropriate circumstances such that the embodiments of the apparatus, methods, and/or articles of manufacture described herein are, for example, capable of operation in other orientations than those illustrated or otherwise described herein.

The terms "couple," "coupled," "couples," "coupling," and the like should be broadly understood and refer to connecting two or more elements mechanically and/or otherwise. Two or more electrical elements may be electrically coupled together, but not be mechanically or otherwise coupled together. Coupling may be for any length of time, e.g., permanent or semi-permanent or only for an instant. "Electrical coupling" and the like should be broadly understood and include electrical coupling of all types. The absence of the word "removably," "removable," and the like near the word "coupled," and the like does not mean that the coupling, etc. in question is or is not removable.

As defined herein, "approximately" can, in some embodiments, mean within plus or minus ten percent of the stated value. In other embodiments, "approximately" can mean within plus or minus five percent of the stated value. In further embodiments, "approximately" can mean within plus or minus three percent of the stated value. In yet other embodiments, "approximately" can mean within plus or minus one percent of the stated value.

The invention relates to a device for detecting heart rate. The device comprises at least one capacitive displacement sensor coupled to a strap. The at least one capacitive displacement sensor comprises two electrodes. The two electrodes comprise an outer transmitting electrode and an inner receiving electrode. The at least one capacitive displacement sensor detects a pulse by producing a signal associated with a distance change between a skin of a wearer of the device and the at least one capacitive displacement sensor.

Some embodiments comprise a system. The system comprises at least one capacitive displacement sensor coupled to a strap. The at least one capacitive displacement sensor comprises two electrodes. The two electrodes comprise an outer transmitting electrode and an inner receiving electrode. The at least one capacitive displacement sensor detects a pulse by producing a signal associated with a distance change between a skin of a wearer of the device and the at least one capacitive displacement sensor. The system further comprises a signal processor receiving one or more signals from the at least one capacitive displacement sensor, the one or more signals comprising information about the one or more pulses, wherein the signal processor detects, from the information, one or more peak pulses of the one or more pulses and determines a heart rate waveform therefrom.

The invention also relates to a method. The method comprises detecting one or more pulses by using at least one capacitive displacement sensor to produce a signal that is related to a change in a distance between the at least one capacitive displacement sensor and a skin of a wearer of the at least one capacitive displacement sensor. The at least one capacitive displacement sensor comprises two electrodes. The two electrodes comprise an outer transmitting electrode and an inner receiving electrode. The method further comprises converting one or more signals from the at least one capacitive displacement sensor into a heart rate waveform, the one or more signals comprising information about the one or more pulses.

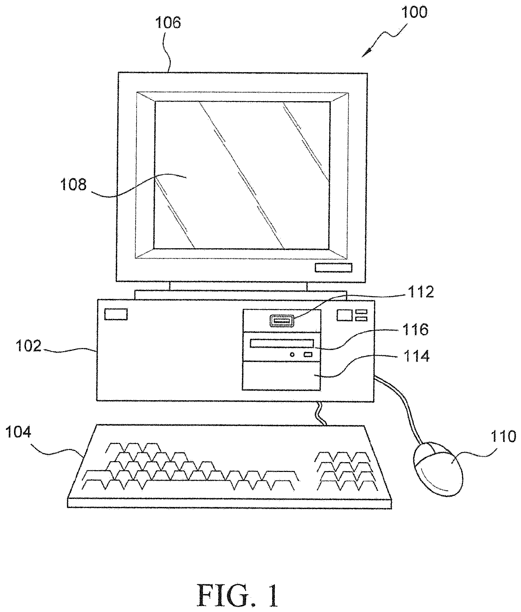

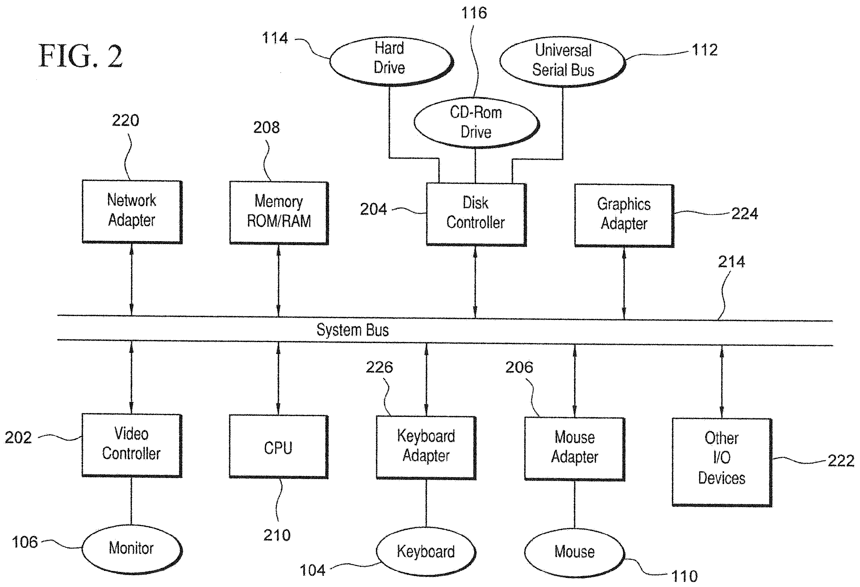

Turning to the drawings, FIG. 1 illustrates an exemplary embodiment of a computer system 100, all of which or a portion of which can be suitable for (i) implementing part or all of one or more embodiments of the techniques, methods, and systems and/or (ii) implementing and/or operating part or all of one or more embodiments of the memory storage modules described herein. As an example, a different or separate one of a chassis 102 (and its internal components) can be suitable for implementing part or all of one or more embodiments of the techniques, methods, and/or systems described herein. Furthermore, one or more elements of computer system 100 (e.g., a monitor 106, a keyboard 104, and/or a mouse 110, etc.) also can be appropriate for implementing part or all of one or more embodiments of the techniques, methods, and/or systems described herein. Computer system 100 can comprise chassis 102 containing one or more circuit boards (not shown), a Universal Serial Bus (USB) port 112, a Compact Disc Read-Only Memory (CD-ROM) and/or Digital Video Disc (DVD) drive 116, and a hard drive 114. A representative block diagram of the elements included on the circuit boards inside chassis 102 is shown in FIG. 2. A central processing unit (CPU) 210 in FIG. 2 is coupled to a system bus 214 in FIG. 2. In various embodiments, the architecture of CPU 210 can be compliant with any of a variety of commercially distributed architecture families.

Continuing with FIG. 2, system bus 214 also is coupled to a memory storage unit 208, where memory storage unit 208 can comprise (i) volatile (e.g., transitory) memory, such as, for example, read only memory (ROM) and/or (ii) non-volatile (e.g., non-transitory) memory, such as, for example, random access memory (RAM). The non-volatile memory can be removable and/or non-removable non-volatile memory. Meanwhile, RAM can include dynamic RAM (DRAM), static RAM (SRAM), etc. Further, ROM can include mask-programmed ROM, programmable ROM (PROM), one-time programmable ROM (OTP), erasable programmable read-only memory (EPROM), electrically erasable programmable ROM (EEPROM) (e.g., electrically alterable ROM (EAROM) and/or flash memory), etc. The memory storage module(s) of the various embodiments disclosed herein can comprise memory storage unit 208, an external memory storage drive (not shown), such as, for example, a USB-equipped electronic memory storage drive coupled to universal serial bus (USB) port 112 (FIGs. 1-2), hard drive 114 (FIGs. 1-2), a CD-ROM and/or DVD for use with a CD-ROM and/or DVD drive 116 (FIGs. 1-2), floppy disk for use with a floppy disk drive (not shown), an optical disc (not shown), a magnetooptical disc (now shown), magnetic tape (not shown), etc. Further, non-volatile or non-transitory memory storage module(s) refer to the portions of the memory storage module(s) that are non-volatile (e.g., non-transitory) memory.

In various examples, portions of the memory storage module(s) of the various embodiments disclosed herein (e.g., portions of the non-volatile memory storage module(s)) can be encoded with a boot code sequence suitable for restoring computer system 100 (FIG. 1) to a functional state after a system reset. In addition, portions of the memory storage module(s) of the various embodiments disclosed herein (e.g., portions of the non-volatile memory storage module(s)) can comprise microcode such as a Basic Input-Output System (BIOS) operable with computer system 100 (FIG. 1). In the same or different examples, portions of the memory storage module(s) of the various embodiments disclosed herein (e.g., portions of the non-volatile memory storage module(s)) can comprise an operating system, which can be a software program that manages the hardware and software resources of a computer and/or a computer network. The BIOS can initialize and test components of computer system 100 (FIG. 1) and load the operating system. Meanwhile, the operating system can perform basic tasks such as, for example, controlling and allocating memory, prioritizing the processing of instructions, controlling input and output devices, facilitating networking, and managing files. Exemplary operating systems can comprise one of the following: (i) Microsoft^{®} Windows^{®} operating system (OS) by Microsoft Corp. of Redmond, Washington, United States of America, (ii) Mac^{®} OS X by Apple Inc. of Cupertino, California, United States of America, (iii) UNIX^{®} OS, and (iv) Linux^{®} OS. Further exemplary operating systems can comprise one of the following: (i) the iOS^{®} operating system by Apple Inc. of Cupertino, California, United States of America, (ii) the Blackberry^{®} operating system by Research In Motion (RIM) of Waterloo, Ontario, Canada, (iii) the WebOS operating system by LG Electronics of Seoul, South Korea, (iv) the Android^{™} operating system developed by Google, of Mountain View, California, United States of America, (v) the Windows Mobile^{™} operating system by Microsoft Corp. of Redmond, Washington, United States of America, or (vi) the Symbian^{™} operating system by Accenture PLC of Dublin, Ireland.

As used herein, "processor" and/or "processing module" means any type of computational circuit, such as but not limited to a microprocessor, a microcontroller, a controller, a complex instruction set computing (CISC) microprocessor, a reduced instruction set computing (RISC) microprocessor, a very long instruction word (VLIW) microprocessor, a graphics processor, a digital signal processor, or any other type of processor or processing circuit capable of performing the desired functions. In some examples, the one or more processing modules of the various embodiments disclosed herein can comprise CPU 210.

In the depicted embodiment of FIG. 2, various I/O devices such as a disk controller 204, a graphics adapter 224, a video controller 202, a keyboard adapter 226, a mouse adapter 206, a network adapter 220, and other I/O devices 222 can be coupled to system bus 214. Keyboard adapter 226 and mouse adapter 206 are coupled to keyboard 104 (FIGs. 1-2) and mouse 110 (FIGs. 1-2), respectively, of computer system 100 (FIG. 1). While graphics adapter 224 and video controller 202 are indicated as distinct units in FIG. 2, video controller 202 can be integrated into graphics adapter 224, or vice versa in other embodiments. Video controller 202 is suitable for monitor 106 (FIGs. 1-2) to display images on a screen 108 (FIG. 1) of computer system 100 (FIG. 1). Disk controller 204 can control hard drive 114 (FIGs. 1-2), USB port 112 (FIGs. 1-2), and CD-ROM drive 116 (FIGs. 1-2). In other embodiments, distinct units can be used to control each of these devices separately.

Network adapter 220 can be suitable to connect computer system 100 (FIG. 1) to a computer network by wired communication (e.g., a wired network adapter) and/or wireless communication (e.g., a wireless network adapter). In some embodiments, network adapter 220 can be plugged or coupled to an expansion port (not shown) in computer system 100 (FIG. 1). In other embodiments, network adapter 220 can be built into computer system 100 (FIG. 1). For example, network adapter 220 can be built into computer system 100 (FIG. 1) by being integrated into the motherboard chipset (not shown), or implemented via one or more dedicated communication chips (not shown), connected through a PCI (peripheral component interconnector) or a PCI express bus of computer system 100 (FIG. 1) or USB port 112 (FIG. 1).

Returning now to FIG. 1, although many other components of computer system 100 are not shown, such components and their interconnection are well known to those of ordinary skill in the art. Accordingly, further details concerning the construction and composition of computer system 100 and the circuit boards inside chassis 102 are not discussed herein.

Meanwhile, when computer system 100 is running, program instructions (e.g., computer instructions) stored on one or more of the memory storage module(s) of the various embodiments disclosed herein can be executed by CPU 210 (FIG. 2). At least a portion of the program instructions, stored on these devices, can be suitable for carrying out at least part of the techniques and methods described herein.

Further, although computer system 100 is illustrated as a desktop computer in FIG. 1, there can be examples where computer system 100 may take a different form factor while still having functional elements similar to those described for computer system 100. In some embodiments, computer system 100 may comprise a single computer, a single server, or a cluster or collection of computers or servers, or a cloud of computers or servers. Typically, a cluster or collection of servers can be used when the demand on computer system 100 exceeds the reasonable capability of a single server or computer. In certain embodiments, computer system 100 may comprise a portable computer, such as a laptop computer. In certain other embodiments, computer system 100 may comprise a mobile electronic device, such as a smartphone. In certain additional embodiments, computer system 100 may comprise an embedded system.

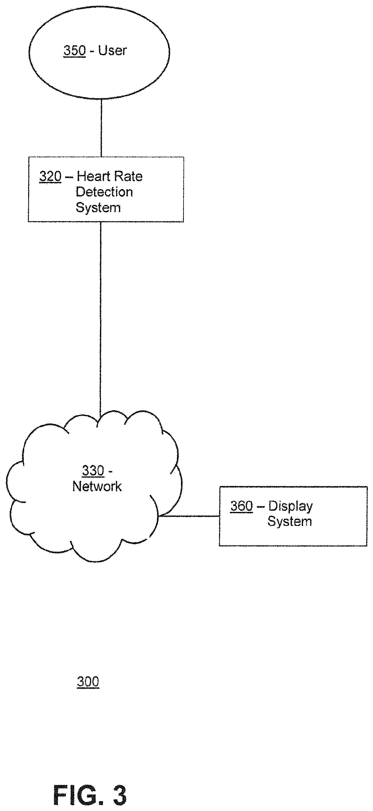

Skipping ahead now in the drawings, FIG. 3 illustrates a representative block diagram of a system 300, according to an embodiment. System 300 is merely exemplary and embodiments of the system are not limited to the embodiments presented herein. System 300 can be employed in many different embodiments or examples not specifically depicted or described herein. In some embodiments, certain elements or modules of system 300 can perform various methods and/or activities of those methods. In these or other embodiments, the methods and/or the activities of the methods can be performed by other suitable elements or modules of system 300.

Generally, therefore, system 300 can be implemented with hardware and/or software, as described herein. In some embodiments, part or all of the hardware and/or software can be conventional, while in these or other embodiments, part or all of the hardware and/or software can be customized (e.g., optimized) for implementing part or all of the functionality of system 300 described herein.

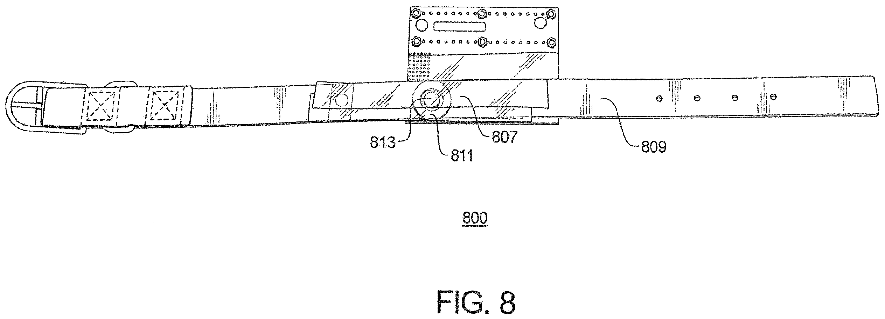

In a number of embodiments, system 300 can comprise a heart rate detection system 320 and a display system 360. In some embodiments, heart rate detection system 320 and display system 360 can each be a computer system 100 (FIG. 1), as described above, and can each be a single computer, a single server, or a cluster or collection of computers or servers. In many embodiments, heart rate detection system 320 can comprise device 800 (FIG. 8).

In many embodiments, heart rate detection system 320 and/or display system 360 can each comprise one or more input devices (e.g., one or more keyboards, one or more keypads, one or more pointing devices such as a computer mouse or computer mice, one or more touchscreen displays, a microphone, etc.), and/or can each comprise one or more display devices (e.g., one or more monitors, one or more touch screen displays, projectors, etc.). In these or other embodiments, one or more of the input device(s) can be similar or identical to keyboard 104 (FIG. 1) and/or a mouse 110 (FIG. 1). Further, one or more of the display device(s) can be similar or identical to monitor 106 (FIG. 1) and/or screen 108 (FIG. 1). The input device(s) and the display device(s) can be coupled to the processing module(s) and/or the memory storage module(s) of heart rate detection system 320 and/or display system 360 in a wired manner and/or a wireless manner, and the coupling can be direct and/or indirect, as well as locally and/or remotely. As an example of an indirect manner (which may or may not also be a remote manner), a keyboard-video-mouse (KVM) switch can be used to couple the input device(s) and the display device(s) to the processing module(s) and/or the memory storage module(s). In some embodiments, the KVM switch also can be part of heart rate detection system 320 and/or display system 360. In a similar manner, the processing module(s) and the memory storage module(s) can be local and/or remote to each other.

In many embodiments, heart rate detection system 320 and/or display system 360 can be configured to communicate with one or more user computers (not shown). In some embodiments, heart rate detection system and/or display system 360 can communicate or interface (e.g. interact) with one or more customer computers through a network 330. In some embodiments, network 330 can be an internet, or an intranet that is not open to the public. Accordingly, in many embodiments, heart rate detection system 320 and/or display system 360 (and/or the software used by such systems) can refer to a back end of system 300 operated by an operator and/or administrator of system 300, and user comptuers (and/or the software used by such systems) can refer to a front end of system 300 used by one or more user 350, respectively. In these or other embodiments, the operator and/or administrator of system 300 can manage system 300, the processing module(s) of system 300, and/or the memory storage module(s) of system 300 using the input device(s) and/or display device(s) of system 300.

Meanwhile, in many embodiments, heart rate detection system 320 and/or display system 360 also can be configured to communicate with one or more databases. The one or more databases can be stored on one or more memory storage modules (e.g., non-transitory memory storage module(s)), which can be similar or identical to the one or more memory storage module(s) (e.g., non-transitory memory storage module(s)) described above with respect to computer system 100 (FIG. 1). Also, in some embodiments, for any particular database of the one or more databases, that particular database can be stored on a single memory storage module of the memory storage module(s), and/or the non-transitory memory storage module(s) storing the one or more databases or the contents of that particular database can be spread across multiple ones of the memory storage module(s) and/or non-transitory memory storage module(s) storing the one or more databases, depending on the size of the particular database and/or the storage capacity of the memory storage module(s) and/or non-transitory memory storage module(s).

The one or more databases can each comprise a structured (e.g., indexed) collection of data and can be managed by any suitable database management systems configured to define, create, query, organize, update, and manage database(s). Exemplary database management systems can include MySQL (Structured Query Language) Database, PostgreSQL Database, Microsoft SQL Server Database, Oracle Database, SAP (Systems, Applications, & Products) Database, and IBM DB2 Database.

Meanwhile, communication between heart rate detection system 320, display system 360, and/or the one or more databases can be implemented using any suitable manner of wired and/or wireless communication. Accordingly, system 300 can comprise any software and/or hardware components configured to implement the wired and/or wireless communication. Further, the wired and/or wireless communication can be implemented using any one or any combination of wired and/or wireless communication network topologies (e.g., ring, line, tree, bus, mesh, star, daisy chain, hybrid, etc.) and/or protocols (e.g., personal area network (PAN) protocol(s), local area network (LAN) protocol(s), wide area network (WAN) protocol(s), cellular network protocol(s), powerline network protocol(s), etc.). Exemplary PAN protocol(s) can comprise Bluetooth, Zigbee, Wireless Universal Serial Bus (USB), Z-Wave, etc.; exemplary LAN and/or WAN protocol(s) can comprise Institute of Electrical and Electronic Engineers (IEEE) 802.3 (also known as Ethernet), IEEE 802.11 (also known as WiFi), etc.; and exemplary wireless cellular network protocol(s) can comprise Global System for Mobile Communications (GSM), General Packet Radio Service (GPRS), Code Division Multiple Access (CDMA), Evolution-Data Optimized (EV-DO), Enhanced Data Rates for GSM Evolution (EDGE), Universal Mobile Telecommunications System (UMTS), Digital Enhanced Cordless Telecommunications (DECT), Digital AMPS (IS-136/Time Division Multiple Access (TDMA)), Integrated Digital Enhanced Network (iDEN), Evolved HighSpeed Packet Access (HSPA+), Long-Term Evolution (LTE), WiMAX, etc. The specific communication software and/or hardware implemented can depend on the network topologies and/or protocols implemented, and vice versa. In many embodiments, exemplary communication hardware can comprise wired communication hardware including, for example, one or more data buses, such as, for example, universal serial bus(es), one or more networking cables, such as, for example, coaxial cable(s), optical fiber cable(s), and/or twisted pair cable(s), any other suitable data cable, etc. Further exemplary communication hardware can comprise wireless communication hardware including, for example, one or more radio transceivers, one or more infrared transceivers, etc. Additional exemplary communication hardware can comprise one or more networking components (e.g., modulator-demodulator components, gateway components, etc.)

Turning ahead in the drawings, FIG. 8 illustrates a device 800 for detecting heart rate. The device 800 comprises at least one capacitive displacement sensor 807 coupled to a strap 809. In some embodiments, strap 809 can comprise at least one of a harness, a belt, a vest, a collar, or a garment. In some embodiments, strap 809 can be configured to be worn over the skin of an animal (e.g., a vest worn by a dog). The at least one capacitive displacement sensor 807 comprises two electrodes. In some embodiments, the at least one capacitive displacement sensor can comprise more than two electrodes. The two electrodes comprise an outer transmitting electrode 811 and an inner receiving electrode 813. In some embodiments, the two electrodes can comprise a first electrode configured to transmit one or more signals and a second electrode configured to receive one or more signals. The outer transmitting electrode 811 and inner receiving electrode 813 are in concentric arrangement. In some embodiments, outer transmitting electrode 811 and inner receiving electrode 813 can be concentric circles. In various embodiments, device 800 can comprise more than one capacitive displacement sensors that can be placed at approximately equidistant intervals across strap 809. In some embodiments, at least one capacitive displacement sensor 807 can further comprise a ground electrode. In a number of embodiments, at least one capacitive displacement sensor 807 can comprise at least one outer transmitting electrode 811 and more than one inner receiving electrode 813.

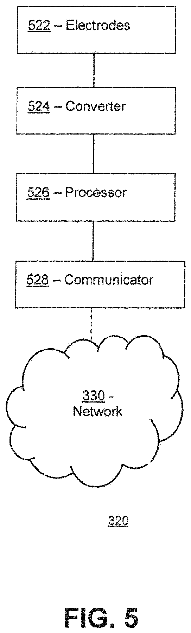

The at least one capacitive displacement sensor 807 detects a pulse by producing a signal associated with a distance change between a skin of a wearer of the device and the at least one capacitive displacement sensor. In some embodiments, at least one capacitive displacement sensor 807 can detect a heart rate, a heart beat-to-beat interval, a respiration rate, and/or a breath-to-breath interval by producing a signal associated with a distance change between a skin of a wearer (e.g., user 350 (FIG. 3) or a pet of user 350 (FIG. 3)) of device 800 and at least one capacitive displacement sensor 807. In some embodiments, the wearer can be a dog or a cat, or other similar pet and/or animal. In some embodiments, heart rate variability can be determined by measuring a time between heartbeats of the wearer of device 800. As discussed further below, in many embodiments, a processor (e.g., processor 526 (FIG. 5) can be used to process the signal produced by at least one capacitive displacement sensor 807 in order to extract information associated with the heart rate and/or breathing of the wearer (e.g. user 350 (FIG. 3)) (e.g., heart rate, heart beat-to-beat interval, respiration rate, and/or breath-to-breath interval).

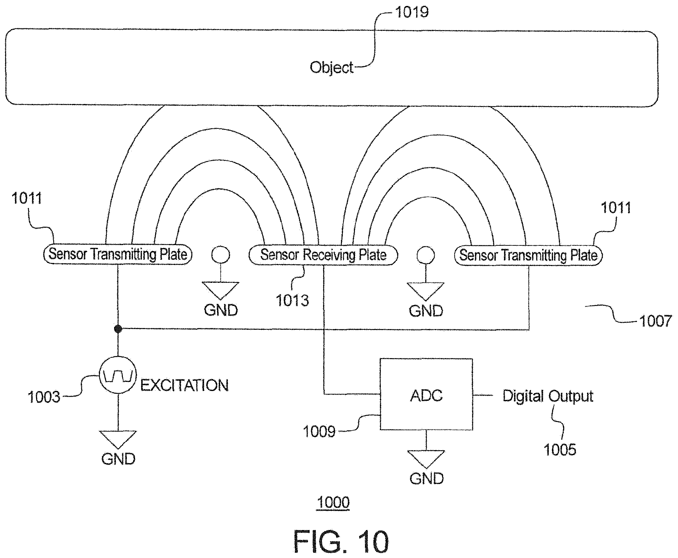

In many embodiments, the at least one capacitive displacement sensor can measure the change in a distance between the at least one capacitive displacement sensor and a skin of a wearer of the at least one capacitive displacement sensor by capacitive proximity sensing. Turning to FIG. 10, device 1000 is a heart rate detection device. In some embodiments, device 1000 can be similar to device 800 (FIG. 8). Device 1000 comprises at least one capacitive displacement sensor 1007. The at least one capacitive displacement sensor 1007 comprises at least one outer transmitting electrode 1011 and at least one inner receiving electrode 1013. In many embodiments, outer transmitting electrode 1011 can be similar to outer transmitting electrode 811 (FIG. 8) and inner transmitting electrode 1013 can be similar to inner transmitting electrode 813 (FIG. 8).

In some embodiments, a power source can supply AC excitation voltage 1003 applied to outer transmitting electrode 1011. In some embodiments, a capacitance to digital converter can comprise the power source for AC excitation voltage 1003 and an analog-to-digital converter 1009. In many embodiments, the capacitance resolution of the capacitance to digital converter can be approximately 164 femtofarads. In various embodiments, power consumption of the capacitance to digital converter can be approximately 700 microamps at 3.3 volts. In many embodiments, inner receiving electrode 1013 can receive a signal from outer transmitting electrode 1011 due to capacitive coupling between outer transmitting electrode 1011 and inner receiving electrode 1013. As an object 1019 (e.g., skin of the wearer or user raised during a heart beat or pulse) approaches outer transmitting electrode 1011 of device and inner receiving electrode 1013, capacitive coupling can shunt the signal away from inner receiving electrode 1013. In many embodiments, the shunting of the signal away from inner receiving electrode 1013 can cause the signal amplitude to be reduced. In a number of embodiments, a signal can be produced based at least in part on a change in distance due in part to the shunting of the signal away from inner receiving electrode 1013. In some embodiments, the change in distance can be approximately 0.01 centimeter (cm) to approximately 3 cm. In some embodiments, a pulse of the carotid artery can cause dilation of the surface of the artery, which can result in an approximately 0.01 cm raise on a skin of an animal. In many embodiments, the change of distance can depend at least in part on a size of the animal, a breed of the animal, and/or a type of coat of the animal. In many embodiments, the signal produced can be converted into a digital output signal 1005.

Returning to FIG. 8, in a number of embodiments, at least one capacitive displacement sensor 807 can be placed at a neck of a wearer (e.g., user 350 (FIG. 3)) and approximate to a carotid artery of the wearer (e.g., user 350 (FIG. 3)). In many embodiments, at least one capacitive displacement sensor 807 does not touch a skin of the wearer. In many embodiments, at least one capacitive displacement sensor 807 is a non-contact displacement sensor. In some embodiments, at least one capacitive displacement sensor 807 can be placed at a chest of the wearer (e.g., user 350 (FIG. 3)).

In various embodiments, device 800 can further comprise an array of capacitive sensors. In many embodiments, the array of capacitive sensors can comprise at least one capacitive displacement sensor 807. In some embodiments, the array of capacitive sensors can comprise at least two capacitive displacement sensors. In some embodiments, the array of capacitive sensors can comprise a linear array. In some embodiments, the array of capacitive sensors can comprise a 2-dimensional array (e.g., on an inside surface of a vest).

In many embodiments, device 800 can further comprise one or more one or more inertial sensors. In some embodiment, the one or more inertial sensors can sense motion caused by a movement of the wearer (e.g., user 350 (FIG. 3)) of the device. In some embodiments, the movement of the wearer (e.g., user 350 (FIG. 3)) can be associated with the wearer walking, running, or any other body motion that is not associated with breathing. In some embodiments, at least a portion of the one or more inertial sensors can be co-located with at least one capacitive displacement sensor 807. As an example, in some embodiments, at least one capacitive displacement sensor 807 and the at least the portion of the one or more inertial sensors can be located on a left side of the chest of the wearer (e.g., user 350 (FIG. 3)), and/or another portion of the one or more inertial sensors and a different capacitive displacement sensor can be located on a right side of the chest of the wearer (e.g., user 350 (FIG. 3)) to balance the weight of the strap across the wearer.

Turning back to heart rate detection system 320 in FIG. 5, in many embodiments, the output signal from at least one capacitive displacement sensor 807 (FIG. 8) can be a waveform. In many embodiments, the waveform can comprise information associated with the heart rate and/or breathing of the wearer (e.g. user 350 (FIG. 3)) (e.g., heart rate, heart beat-to-beat interval, respiration rate, and/or breath-to-breath interval). In some embodiments, heart rate detection system 320 can comprise one or more electrodes 522 (e.g., outer transmitting electrode 811 and inner receiving electrode 813 (FIG. 8)), converter 524, processor 526, and communicator 528.

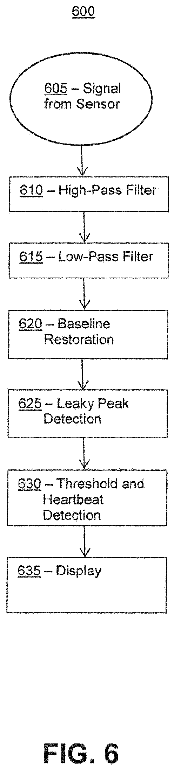

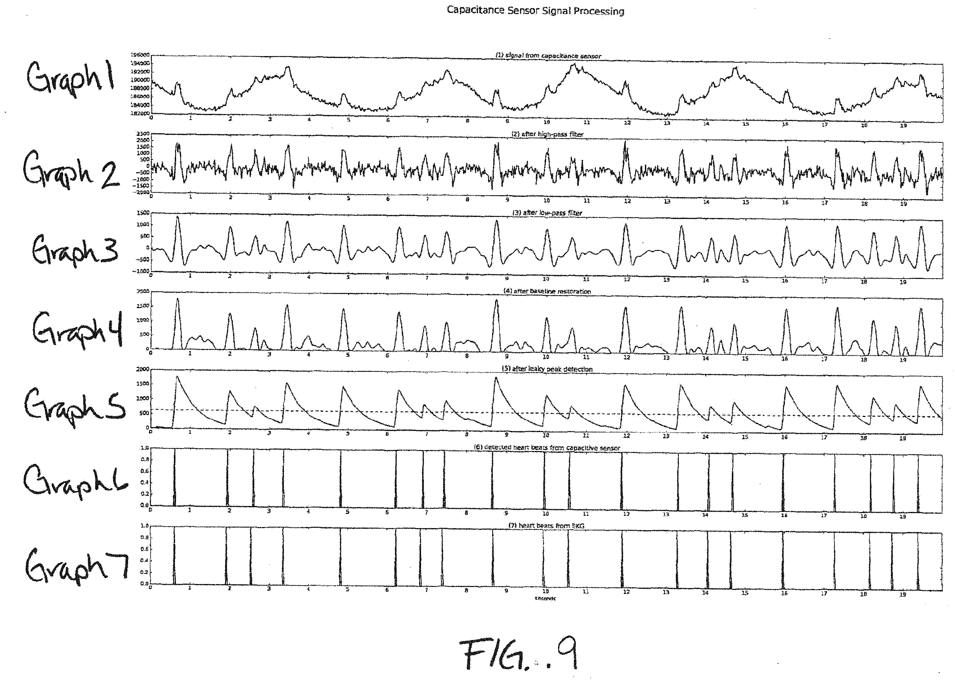

In some embodiments, heart rate detection system 320 (FIG. 5) can process the signal produced by at least one capacitive displacement sensor 807 (FIG. 8) according to a method 600 (FIG. 6). Discussing FIGs. 6 and 9 together, in many embodiments, method 600 can process the signal produced by at least one capacitive displacement sensor 807 (FIG. 8).

FIG. 9 illustrates 7 graphs representing approximately 20 seconds of data captured by at least one capacitive displacement sensor 807 (FIG. 8). In many embodiments, the output signal from the at least one capacitive displacement sensor 807 (FIG. 8) comprises a digital (e.g., numeric) representation of a capacitance value sensed by the electrodes (e.g., outer transmitting electrode 811 and inner receiving electrode 813 (FIG. 8) as sent in activity 605 (FIG. 6). This output signal can be raw or unprocessed and comprises information related to the breathing and heart beats of the dog. Graph 1 in FIG. 9 illustrates the output of activity 605.

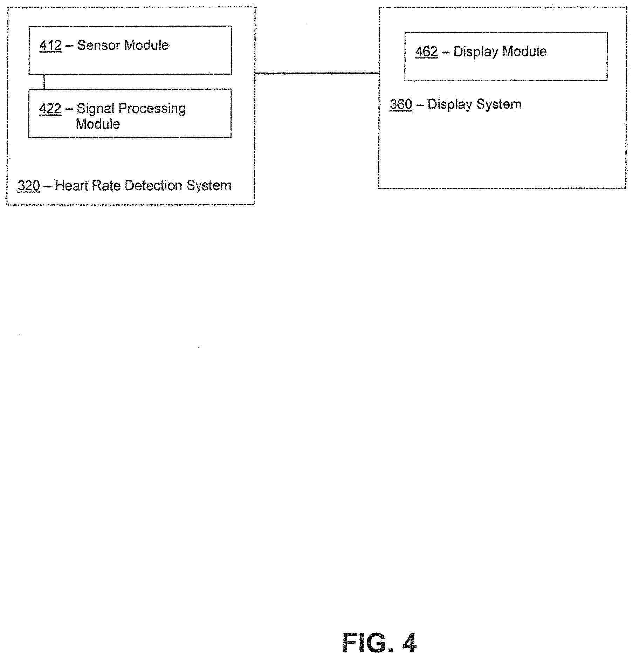

Turning back in the drawings, FIG. 6 illustrates a flow chart for a method 600, according to an embodiment. Method 600 is merely exemplary and is not limited to the embodiments presented herein. Method 600 can be employed in many different embodiments or examples not specifically depicted or described herein. In some embodiments, the heart rate detecting device 800 (FIG. 8) and/or device 1000 (FIG. 10) can be used in association with method 600. In some embodiments, the activities of method 600 can be performed in the order presented. In other embodiments, the activities of method 600 can be performed in any suitable order. In still other embodiments, one or more of the activities of method 600 can be combined or skipped. In many embodiments, system 300 (FIG. 3) can be suitable to perform method 600 and/or one or more of the activities of method 600. In these or other embodiments, one or more of the activities of method 600 can be implemented as one or more computer instructions configured to run at one or more processing modules and configured to be stored at one or more non-transitory memory storage modules 412, 422, and/or 462 (FIG. 4). Such non-transitory memory storage modules can be part of a computer system such as heart rate device system 320 (FIGs. 3 & 4) and/or display system 360 (FIGs. 3 & 4). The processing module(s) can be similar or identical to the processing module(s) described above with respect to computer system 100 (FIG. 1).

In many embodiments, method 600 can comprise an activity 610 of filtering the signal from activity 605 with a high-pass filter. In some embodiments, the high-pass filter can comprise a cutoff frequency of approximately 1 Hertz (Hz). In many embodiments, activity 610 can remove slow-moving signals, such as signals caused by respiration, in order to remove an offset from the data produced by activity 605. Graph 2 (FIG. 9) illustrates the output of activity 610.

In various embodiments, method 600 further can comprise an activity 615 of filtering the signal through a low-pass filter. In many embodiments, activity 615 can comprise removing high-frequency noise by using the low-pass filter. In some embodiments, a cutoff frequency of the low-pass filter can be approximately 10 Hz. Graph 3 (FIG. 9) illustrates the output of activity 615. In some embodiments, the sequence of activities 610 and 615 is reversed.

In a number of embodiments, method 600 further can comprise an activity 620 of processing the signal to restore a baseline. In some embodiments, activity 620 can comprise processing the signal to set a numeric baseline of zero. In various embodiments, activity 620 can use a baseline restoration (e.g., DC-restoration) algorithm. In some embodiments, the following algorithm (in PYTHON code) can be used, wherein y_bp is the input signal and y_dcr is the output signal:

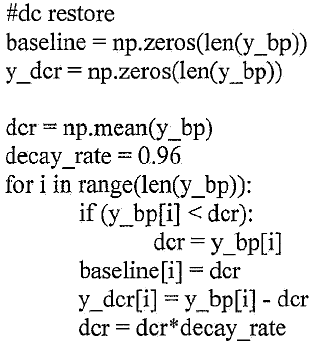

In some embodiments, this processing can emphasize one or more peaks in the signal that can be due to heartbeats. Graph 4 (FIG. 9) illustrates the output of activity 620.

In a number of embodiments, method 600 further can comprise an activity 625 to detect one or more heart beats and reject noise by processing the signal with a "leaky peak detection" algorithm. In many embodiments, following algorithm (in PYTHON code) can be used, where y_dcr is the input and y_pdl is the output:

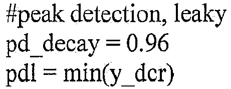

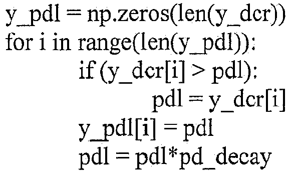

Graph 5 (FIG. 9) illustrates the output of activity 625.

In some embodiments, method 600 also can comprise activity 630 of determining a threshold and detecting a heart beat. In many embodiments, to detect heart beats from the signal of graph 5 (FIG. 9), activity 630 can comprise determining a threshold value as the average of the signal. The threshold value can be shown as the dashed line in graph 5 (FIG. 9). In many embodiments, a heart beat can be detected when the signal has a rising edge that crosses the threshold value (e.g., dashed line). Graph 6 (FIG. 9) illustrates the output of activity 630. As shown in graph 6 (FIG. 9), each vertical spike indicates a detected heartbeat. The signal in graph 6 (FIG. 9) can be used to measure beat-to-beat variation. In some embodiments, activity 600 also can comprise activity 635 of displaying the heart beat.

In many embodiments, to verify that the heart beat detecting device (e.g. device 800 (FIG. 8) is correctly detecting heart beats from the user (e.g., user 350 (FIG. 3), one or more heart beats from an electrocardiogram based (EKG-based) heart rate monitor can be collected simultaneously with the capacitive signal. The one or more heart beats recorded from the EKG-based monitor are illustrated in graph 7 (FIG. 9). In the case of this approximately 20-second data set, the heart beat timing detected by the heart beat detecting device (e.g. device 800 (FIG. 8) in graph 6 corresponds to the heart beat timing from the EKG-based monitor in graph 7 (FIG. 9).

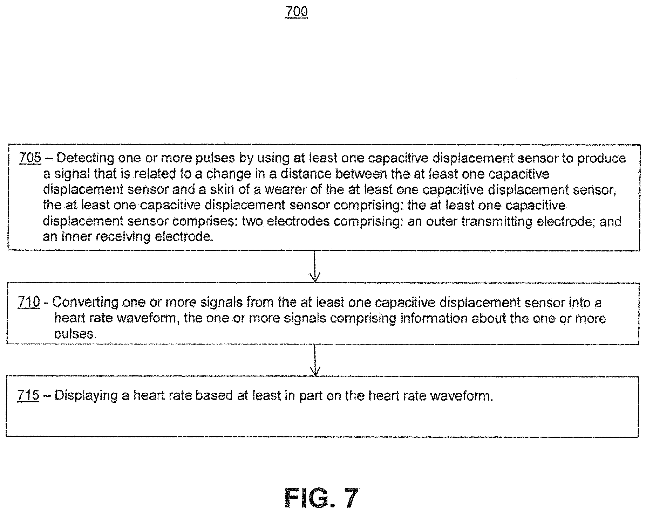

Turning ahead in the drawings, FIG. 7 illustrates a flow chart for a method 700, according to an embodiment. Method 700 is merely exemplary and is not limited to the embodiments presented herein. Method 700 can be employed in many different embodiments or examples not specifically depicted or described herein. In some embodiments, the heart rate detecting device 800 (FIG. 8) can be used in association with method 700. In some embodiments, the activities of method 700 can be performed in the order presented. In other embodiments, the activities of method 700 can be performed in any suitable order. In still other embodiments, one or more of the activities of method 700 can be combined or skipped. In many embodiments, system 300 (FIG. 3) can be suitable to perform method 700 and/or one or more of the activities of method 700. In these or other embodiments, one or more of the activities of method 700 can be implemented as one or more computer instructions configured to run at one or more processing modules and configured to be stored at one or more non-transitory memory storage modules 412, 422, and/or 462 (FIG. 4). Such non-transitory memory storage modules can be part of a computer system such as heart rate device system 320 (FIGs. 3 & 4) and/or display system 360 (FIGs. 3 & 4). The processing module(s) can be similar or identical to the processing module(s) described above with respect to computer system 100 (FIG. 1).

Method 700 comprises an activity 705 of detecting one or more pulses by using at least one capacitive displacement sensor to produce a signal that is related to a change in a distance between the at least one capacitive displacement sensor and a skin of a wearer of the at least one capacitive displacement sensor. The at least one capacitive displacement sensor comprises two electrodes comprising an outer transmitting electrode (e.g. outer transmitting electrode 811 (FIG. 8)) and an inner receiving electrode (e.g., inner receiving electrode 813 (FIG. 8)). In some embodiments, the at least one capacitive displacement sensor can be similar to at least one capacitive displacement sensor 807 (FIG. 8). In various embodiments, the at least one capacitive displacement sensor can be attached to a strap (e.g., strap 809 (FIG. 8)). In some embodiments, the two electrodes can comprise a first electrode configured to transmit one or more signals and a second electrode configured to receive one or more signals. The outer transmitting electrode and the inner receiving electrode are in concentric arrangement. In some embodiments, the outer transmitting electrode and the inner receiving electrode can be concentric circles. In various embodiments, the heart beat detecting device (e.g., device 800 (FIG. 8)) can comprise more than one capacitive displacement sensors that can be placed at approximately equidistant intervals across the strap. In some embodiments, the at least one capacitive displacement sensor can further comprise a ground electrode. In a number of embodiments, the at least one capacitive displacement sensor can comprise at least one outer transmitting electrode and more than one inner receiving electrode.

In one embodiment, one electrode could be a ground electrode, such as a metal wire that goes all the way around the collar or the collar itself if the collar were made of an electrically conductive material or a fabric with electrically conductive fibers in it. In this embodiment, the active electrode would be a metal patch located on the inside of the collar with an insulator separating it from the main collar strap ground electrode.

In another embodiments, the capacitance between the electrodes is not sensed by making one electrode transmit and the other receive. One common way to do this is to make the capacitance of one electrode relative to ground (or the strap) part of an oscillator's resonant circuit. When this is done, the frequency of the oscillation changes as the capacitance between the electrode and ground changes. The oscillator's output then goes to a frequency to voltage converter to get a signal that would look basically the same as that from an AD7746-based sensor. This single-electrode capacitive sensing technique is the principle of the well-known "Theremin" musical instrument, where extremely small changes in capacitance between the "antenna" (a single electrode) and ground affect the pitch of an audio oscillator.

In many embodiments, activity 705 can comprise applying an alternating current (AC) excitation voltage to the outer transmitting electrode as shown in FIG. 10 and discussed above. In some embodiments, detecting the one or more pulses can further comprise using an array of capacitive displacement sensors, the array of capacitive displacement sensors comprising the at least one capacitive displacement sensor. In many embodiments, the array of capacitive sensors can comprise at least one capacitive displacement sensor. In some embodiments, the array of capacitive sensors can comprise at least two capacitive displacement sensors. In some embodiments, the array of capacitive sensors can comprise a linear array. In some embodiments, the array of capacitive sensors can comprise a 2-dimensional array (e.g., on an inside surface of a vest).

The method 700 also comprises an activity 710 of converting one or more signals from the at least one capacitive displacement sensor into a heart rate waveform, the one or more signals comprising information about the one or more pulses. In many embodiments, activity 710 can be similar to method 600 (FIG. 6). In some embodiments, method 700 can further comprise an activity of detecting a motion caused by a movement of the wearer of the at least one capacitive displacement sensor by using one or more inertial sensors. In some embodiments, method 700 can further comprise an activity of filtering the heart rate waveform by removing data related to the motion caused by the movement of the wearer of the at least one capacitive displacement sensor. In some embodiments, method 700 also can comprise an activity 715 of displaying a heart rate based at least in part on the heart rate waveform.

Returning to FIG. 4, FIG. 4 illustrates a block diagram of a portion of system 300 comprising heart rate detection system 320 and/or display system 360, according to the embodiment shown in FIG. 3. Each of heart rate detection system 320 and/or display system 360 are merely exemplary and are not limited to the embodiments presented herein. Each of heart rate detection system 320 and/or display system 360 can be employed in many different embodiments or examples not specifically depicted or described herein. In some embodiments, certain elements or modules of heart rate detection system 320 and/or display system 360 can perform various procedures, processes, and/or acts. In other embodiments, the procedures, processes, and/or acts can be performed by other suitable elements or modules.

In many embodiments, heart rate detection system 320 can comprise non-transitory memory storage modules 412 and 422, and display module can comprise a non-transitory memory storage module 462. Memory storage module 412 can be referred to as a sensor module 412, and memory storage module 422 can be referred to as a signal processing module 422. Memory storage module 462 can be referred to as a display module 462.

In many embodiments, sensor module 412 can store computing instructions configured to run on one or more processing modules and perform one or more acts related to sensing. In various embodiments, sensor module 412 can store computing instructions configured to run on one or more processing modules and perform one or more acts of methods 700 (FIG. 7) (e.g., activity 705 (FIG. 7)) or one or more acts of method 600 (FIG. 6) (e.g., activity 605 (FIG. 6)). In some embodiments, signal processing module 422 can store computing instructions configured to run on one or more processing modules and perform one or more acts of method 700 (FIG. 7) (e.g., activity 710 (FIG. 7) and/or activities 610, 615, 620, and/or 625 (FIG. 6)).

In some embodiments, display module 462 can store computing instructions configured to run on one or more processing modules and perform one or more acts of method 700 (FIG. 7) (e.g., activity 715 (FIG. 7)) or one or more acts of method 600 (FIG. 6) (e.g., activity 635 (FIG. 6)).

Although a heart rate detection device and related systems and methods has been described above, it will be understood by those skilled in the art that various changes may be made without departing from the scope of the invention which is defined by the claims. Accordingly, the disclosure of embodiments is intended to be illustrative of the scope of the disclosure and is not intended to be limiting. The scope of the invention is limited only to the extent required by the appended claims. For example, to one of ordinary skill in the art, it will be readily apparent that any element of the figures in FIGs. 1-9 may be modified, and that the foregoing discussion of certain of these embodiments does not necessarily represent a complete description of all possible embodiments. For example, one or more of the activities of the FIGs. 6-7 may include different activities and/or be performed by many different modules, in many different orders.

In another embodiment, the disclosure provides a magnetic field sensor, such as ST micro LSM303 3-axis compass integrated circuit, mounted with the capacitative sensor. The 3-axis compass signals can also be used to detect a dog's motion. The information about the dog's motion can be used to identify and remove motion artifacts in the capacittaive sensor signal.

## Claims

1. A device (800, 1000) for detecting a heart rate, the device comprising at least one capacitive displacement sensor (807) coupled to a strap (809), wherein the at least one capacitive displacement sensor comprises at least two electrodes (522), and the at least one capacitive displacement sensor detects a pulse by producing a signal associated with a distance change between a skin of a wearer of the device and the at least one capacitive displacement sensor, wherein the at least one capacitive displacement sensor comprises an outer transmitting electrode (811) and an inner receiving electrode (813), wherein the outer transmitting electrode and the inner receiving electrode are in concentric arrangement.

2. The device of claim 1, further comprising one or more inertial sensors, wherein the one or more inertial sensors sense motion caused by a movement of the wearer of the device.

3. A system (320) for determining a heart rate, the system comprising:
i) the device of claim 1; and
ii) a signal processor (526) receiving one or more signals from the at least one capacitive displacement sensor, the one or more signals comprising information about the one or more pulses, wherein the signal processor detects, from the information, one or more peak pulses of the one or more pulses and determines a heart rate waveform therefrom.

4. The device of claim 1 or the system of claim 3, wherein the strap comprises at least one of a harness; a belt; a vest; or a collar.

5. The device of claim 1 or the system of claim 3, further comprising: an array of capacitive displacement sensors, the array of capacitive displacement sensors comprising the at least one capacitive displacement sensor.

6. The system of claim 3, further comprising: one or more inertial sensors, wherein the one or more inertial sensors sense motion caused by a movement of the wearer of the device.

7. A method (700) for determining a heart rate comprising:
detecting (705) one or more pulses by using at least one capacitive displacement sensor (807) to produce a signal that is related to a change in a distance between the at least one capacitive displacement sensor and a skin of a wearer of the at least one capacitive displacement sensor, the at least one capacitive displacement sensor comprising two electrodes (522); and
converting (710) one or more signals from the at least one capacitive displacement sensor into a heart rate waveform, the one or more signals comprising information about the one or more pulses,
wherein the two electrodes comprise an outer transmitting electrode (811) and an inner receiving electrode (813), wherein the outer transmitting electrode and inner receiving electrode are in concentric arrangement.

8. The method of claim 7, wherein: at least one capacitive displacement sensor is attached to a strap; and the strap comprises at least one of: a harness; a belt; a vest; or a collar.

9. The method of claim 7, wherein: detecting the one or more pulses further comprises using an array of capacitive displacement sensors, the array of capacitive displacement sensors comprising the at least one capacitive displacement sensor.

10. The device or system of claim 5, or the method of claim 9, wherein: the array of capacitive displacement sensors comprises at least two capacitive displacement sensors.

11. The device, system or method of claim 10, wherein: the at least two capacitive displacement sensors are placed at equidistant intervals across the strap.

12. The method of claim 7, further comprising:
detecting a motion caused by a movement of the wearer of the at least one capacitive displacement sensor by using one or more inertial sensors; and
filtering the heart rate waveform by removing data related to the motion caused by the movement of the wearer of the at least one capacitive displacement sensor.

13. The device of claim 1, the system of claim 3 or the method of claim 7, wherein: the at least one capacitive displacement sensor is placed at a neck of the wearer and approximate to a carotid artery of the wearer.

## Patentansprüche

1. Vorrichtung (800, 1000) zum Erfassen einer Herzfrequenz, die Vorrichtung umfassend mindestens einen kapazitiven Verschiebungssensor (807), der mit einem Gurt (809) gekoppelt ist, wobei der mindestens eine kapazitive Verschiebungssensor mindestens zwei Elektroden (522) umfasst und der mindestens eine kapazitive Verschiebungssensor einen Puls erfasst, durch ein Erzeugen eines Signals, das einer Abstandsänderung zwischen einer Haut eines Trägers der Vorrichtung und dem mindestens einen kapazitiven Verschiebungssensor zugeordnet ist, wobei der mindestens eine kapazitive Verschiebungssensor eine äußere Sendeelektrode (811) und eine innere Empfangselektrode (813) umfasst, wobei die äußere Sendeelektrode und die innere Empfangselektrode konzentrisch angeordnet sind.

2. Vorrichtung nach Anspruch 1, ferner umfassend einen oder mehrere Trägheitssensoren, wobei der eine oder die mehreren Trägheitssensoren eine Bewegung erkennen, die durch eine Bewegung des Trägers der Vorrichtung verursacht wird.

3. System (320) zum Bestimmen einer Herzfrequenz, das System umfassend:
i) die Vorrichtung nach Anspruch 1; und
ii) einen Signalprozessor (526), der ein oder mehrere Signale von dem mindestens einen kapazitiven Verschiebungssensor empfängt, das eine oder die mehreren Signale umfassend Informationen über den einen oder die mehreren Pulse, wobei der Signalprozessor aus den Informationen einen oder mehrere Spitzenpulse des einen oder der mehreren Pulse erfasst und daraus eine Herzfrequenzwellenform bestimmt.

4. Vorrichtung nach Anspruch 1 oder System nach Anspruch 3, wobei der Gurt mindestens eines umfasst von einem Riemen; einem Gürtel; einer Weste; oder einem Halsband.

5. Vorrichtung nach Anspruch 1 oder System nach Anspruch 3, ferner umfassend: eine Anordnung von kapazitiven Verschiebungssensoren, die Anordnung von kapazitiven Verschiebungssensoren umfassend den mindestens einen kapazitiven Verschiebungssensor.

6. System nach Anspruch 3, ferner umfassend: einen oder mehrere Trägheitssensoren, wobei der eine oder die mehreren Trägheitssensoren eine Bewegung erkennen, die durch eine Bewegung des Trägers der Vorrichtung verursacht wird.

7. Verfahren (700) zum Bestimmen einer Herzfrequenz, umfassend:
Erfassen (705) eines oder mehrerer Pulse durch Verwenden mindestens eines kapazitiven Verschiebungssensors (807), um ein Signal zu erzeugen, das sich auf eine Änderung eines Abstands zwischen dem mindestens einen kapazitiven Verschiebungssensor und einer Haut eines Trägers des mindestens einen kapazitiven Verschiebungssensors bezieht, der mindestens eine kapazitive Verschiebungssensor umfassend zwei Elektroden (522); und
Umwandeln (710) eines oder mehrerer Signale von dem mindestens einen kapazitiven Verschiebungssensor in eine Herzfrequenzwellenform, das eine oder die mehreren Signale umfassend Informationen über den einen oder die mehreren Pulse,
wobei die zwei Elektroden eine äußere Sendeelektrode (811) und eine innere Empfangselektrode (813) umfassen, wobei die äußere Sendeelektrode und die innere Empfangselektrode konzentrisch angeordnet sind.

8. Verfahren nach Anspruch 7, wobei: mindestens ein kapazitiver Verschiebungssensor an einem Gurt befestigt ist; und der Gurt mindestens eines umfasst von: einem Riemen; einem Gürtel; einer Weste; oder einem Halsband.

9. Verfahren nach Anspruch 7, wobei: das Erfassen des einen oder der mehreren Pulse ferner das Verwenden einer Anordnung von kapazitiven Verschiebungssensoren umfasst, die Anordnung von kapazitiven Verschiebungssensoren umfassend den mindestens einen kapazitiven Verschiebungssensor.

10. Vorrichtung oder System nach Anspruch 5 oder Verfahren nach Anspruch 9, wobei: die Anordnung von kapazitiven Verschiebungssensoren mindestens zwei kapazitive Verschiebungssensoren umfasst.

11. Vorrichtung, System oder Verfahren nach Anspruch 10, wobei: die mindestens zwei kapazitiven Verschiebungssensoren in gleichabständigen Intervallen über den Gurt verteilt sind.

12. Verfahren nach Anspruch 7, ferner umfassend:
Erfassen einer Bewegung, die durch eine Bewegung des Trägers des mindestens einen kapazitiven Verschiebungssensors verursacht wird, durch Verwenden eines oder mehrerer Trägheitssensoren; und
Filtern der Herzfrequenzwellenform durch ein Entfernen von Daten, die sich auf die Bewegung beziehen, die durch die Bewegung des Trägers des mindestens einen kapazitiven Verschiebungssensors verursacht wird.

13. Vorrichtung nach Anspruch 1, System nach Anspruch 3 oder Verfahren nach Anspruch 7, wobei: der mindestens eine kapazitive Verschiebungssensor an einem Hals des Trägers und in der Nähe einer Halsschlagader des Trägers platziert ist.

## Revendications

1. Dispositif (800, 1000) permettant de détecter une fréquence cardiaque, le dispositif comprenant au moins un capteur de déplacement capacitif (807) couplé à une sangle (809), dans lequel l'au moins un capteur de déplacement capacitif comprend au moins deux électrodes (522), et l'au moins un capteur de déplacement capacitif détecte une impulsion en produisant un signal associé à un changement de distance entre la peau d'un porteur du dispositif et l'au moins un capteur de déplacement capacitif, dans lequel l'au moins un capteur de déplacement capacitif comprend une électrode d'émission externe (811) et une électrode de réception interne (813), dans lequel l'électrode d'émission externe et l'électrode de réception interne sont disposées de manière concentrique.

2. Dispositif selon la revendication 1, comprenant en outre un ou plusieurs capteurs inertiels, dans lequel les un ou plusieurs capteurs inertiels détectent un mouvement causé par un déplacement du porteur du dispositif.

3. Système (320) permettant de déterminer une fréquence cardiaque, le système comprenant :
i) le dispositif selon la revendication 1 ; et
ii) un processeur de signaux (526) recevant un ou plusieurs signaux de l'au moins un capteur de déplacement capacitif, les un ou plusieurs signaux comprenant des informations sur les une ou plusieurs impulsions, dans lequel le processeur de signaux détecte, à partir des informations, une ou plusieurs impulsions de crête des une ou plusieurs impulsions et détermine une forme d'onde de fréquence cardiaque à partir de celles-ci.

4. Dispositif selon la revendication 1 ou système selon la revendication 3, dans lequel la sangle comprend au moins un parmi un harnais ; une ceinture ; un gilet ; et un collier.

5. Dispositif selon la revendication 1 ou système selon la revendication 3, comprenant en outre : un réseau de capteurs de déplacement capacitifs, le réseau de capteurs de déplacement capacitifs comprenant l'au moins un capteur de déplacement capacitif.

6. Système selon la revendication 3, comprenant en outre : un ou plusieurs capteurs inertiels, dans lequel les un ou plusieurs capteurs inertiels détectent un mouvement causé par un déplacement du porteur du dispositif.

7. Procédé (700) permettant de déterminer une fréquence cardiaque comprenant :
la détection (705) d'une ou plusieurs impulsions en utilisant au moins un capteur de déplacement capacitif (807) pour produire un signal qui est relatif à un changement d'une distance entre l'au moins un capteur de déplacement capacitif et la peau d'un porteur de l'au moins un capteur de déplacement capacitif, l'au moins un capteur de déplacement capacitif comprenant deux électrodes (522) ; et
la conversion (710) d'un ou plusieurs signaux provenant de l'au moins un capteur de déplacement capacitif en une forme d'onde de fréquence cardiaque, les un ou plusieurs signaux comprenant des informations sur les une ou plusieurs impulsions,
dans lequel les deux électrodes comprennent une électrode d'émission externe (811) et une électrode de réception interne (813), dans lequel l'électrode d'émission externe et l'électrode de réception interne sont disposées de manière concentrique.

8. Procédé selon la revendication 7, dans lequel : au moins un capteur de déplacement capacitif est fixé à une sangle ; et la sangle comprend au moins un parmi : un harnais ; une ceinture ; un gilet ; et un collier.

9. Procédé selon la revendication 7, dans lequel : la détection des une ou plusieurs impulsions comprend en outre l'utilisation d'un réseau de capteurs de déplacement capacitifs, le réseau de capteurs de déplacement capacitifs comprenant l'au moins un capteur de déplacement capacitif.

10. Dispositif ou système selon la revendication 5, ou procédé selon la revendication 9, dans lequel : le réseau de capteurs de déplacement capacitifs comprend au moins deux capteurs de déplacement capacitifs.

11. Dispositif, système ou procédé selon la revendication 10, dans lequel : les au moins deux capteurs de déplacement capacitifs sont placés à intervalles équidistants en travers de la sangle.

12. Procédé selon la revendication 7, comprenant en outre :
la détection d'un mouvement causé par un déplacement du porteur de l'au moins un capteur de déplacement capacitif à l'aide d'un ou de plusieurs capteurs inertiels ; et
le filtrage de la forme d'onde de la fréquence cardiaque en supprimant des données relatives au mouvement causé par le déplacement du porteur de l'au moins un capteur de déplacement capacitif.

13. Dispositif selon la revendication 1, système selon la revendication 3 ou procédé selon la revendication 7, dans lequel : l'au moins un capteur de déplacement capacitif est placé au niveau du cou du porteur et à proximité de l'artère carotide du porteur.
